# EUROPEAN PATENT SPECIFICATION

(11) **EP 4 201 929 B1**
(45) Date of publication and mention of the grant of the patent: **14.08.2024**
(21) Application number: 21217702.6
(22) Date of filing: 24.12.2021
(51) Int. Cl.: C07D 401/04, A61K 31/498, A61P 35/00, A61P 35/02

(54) **QUINOXALINE THIOREDOXIN REDUCTASE INHIBITORS**
QUINOXALINE THIOREDOXIN-REDUKTASE-INHIBITOREN
INHIBITEURS QUINOXALINE DE THIORÉDOXINE RÉDUCTASE

(43) Date of publication of application: 28.06.2023
(73) Proprietor: geneXplain GmbH, 38302 Wolfenbüttel (DE)
(72) Inventor: KEL, Alexander, Wolfenbüttel (DE); SELIVANOVA, Galina, Stockholm (SE); SHCHEKOTIKHIN, Audrey, Moscow (RU); BURAVCHENKO, Galina, Moscow (RU); KOUTCHEROV, Vladimir, Stockholm (SE); POROIKOV, Vladimir, Balashikha, Moscow Region (RU); ZOUBAREV, Roman, Stockholm (SE); ARNÉR, Elias, Stockholm (SE)
(74) Representative: Forstmeyer, Dietmar

(56) References cited:
- EP-A1- 3 138 565
- WO-A1-2018/146472
- RU-C1- 2 640 304
- HU Y ET AL: "Synthesis and biological evaluation of 3-aryl-quinoxaline-2-carbonitrile 1,4-di-N-oxide derivatives as hypoxic selective anti-tumor agents", MOLECULES, MDPI AG, CH, vol. 17, no. 8, 13 August 2012 (2012-08-13), pages 9683 - 9696, XP002733720, ISSN: 1420-3049, DOI: 10.3390/MOLECULES17089683
- ZARRANZ BELEN ET AL: "Synthesis and antimalarial activity of new 3-arylquinoxaline-2-carbonitrile derivatives", ARZNEIMITTEL-FORSCHUNG DRUG RESEARCH, ECD EDITTIO CANTOR VERLAG , AULENDORF, DE, vol. 55, no. 12, 1 January 2005 (2005-01-01), pages 754 - 761, XP001525908, ISSN: 0004-4172
- BALDERAS-RENTERIA I. ET AL: "Anticancer Drug Design Using Scaffolds of [beta]-Lactams, Sulfonamides, Quinoline, Quinoxaline and Natural Products. Drugs Advances in Clinical Trials", CURRENT MEDICINAL CHEMISTRY, vol. 19, no. 26, 1 September 2012 (2012-09-01), NL, pages 4377 - 4398, XP055918012, ISSN: 0929-8673, DOI: 10.2174/092986712803251593

## Description

The present invention relates to novel derivatives of quinoxaline-2-carbonitrile 1,4-dioxide, which are able to selectively induce apoptosis in cancer cells via inhibiting the enzyme thioredoxin reductase TrxRl and triggering oxidative stress, as well as to medical uses thereof.

Cancer remains the second leading cause of death in the developed countries. Novel anticancer compounds are in high demand, each bursting rapidly onto the market upon approval. Target-specific non-genotoxic anti-cancer drugs are the holy grail of modern pharmaceutical industry [C.H. Wong et al., Biostatistics. 2019 Apr; 20(2): 273-286].

Cancer cells are particularly vulnerable to oxidative stress as a result of inhibition of cytosolic selenoprotein thioredoxin reductase TrxRl (TXNRD1) because of inherently increased oxidative stress as a result of oncogenic signaling, distorted metabolism, impaired mitochondrial function, and other mechanisms. From the therapeutic perspective, it is important to note an opposite effect of TrxR1 inhibition on normal and cancer cells: survival of normal cells or even strengthening them against oxidative stress versus death of cancer cells [E.S.J. Arner, Adv Cancer Res 2017, 136:139-151]. Currently, this is one of the most promising anticancer approaches exploiting cancer vulnerability and anticancer drug development.

Resume of these findings suggest that pharmacological inhibition of TrxR1 function by small-weight compounds would result in selective elimination of tumor cells [R. Gencheva, E.S.J.Amer. Annu Rev Pharmacol Toxicol. 2021, doi: 10.1146/annurev-pharmtox-052220-102509]. This idea is based on a number of observations that cancer cells are uniquely vulnerable to TrxR inhibition by virtue of their increased reliance on detoxifying systems counteracting the ROS overproduction accompanying a cancer-specific deregulated metabolism and increased mitogenic signaling. Consequently, there is a need within this field to provide new substances and methods for use therein, which enable inhibition of enzymatic activity of TrxRl and which are able to induce the ROS-dependent apoptosis in tumor cells. Inhibitors of thioredoxin reductase are disclosed in WO 2018/146472 A1.

It has been the object of the present invention to fulfill the above defined need. This object has been achieved by providing novel derivatives of quinoxaline-2-carbonitrile 1,4-dioxide, which are capable of inhibiting of TrxRl and selectively induce death of tumor cells.

The present invention provides one or more compounds of formula (I): 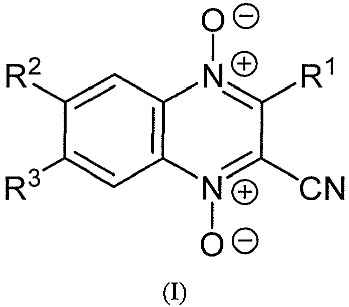 wherein
R¹ is a 2-pyridyl group, a 3-pyridyl group or a 4-pyridyl group, all of which groups may optionally be substituted by 1, 2 or 3 substituents that are independently selected from a C₁₋₆ alkyl group, a C₁₋₆ alkoxy group, a C₁₋₆ alkylthio group and a halogen atom;
R² is a group of formula -NR⁴R⁵, and R³ is a halogen atom; or
R² is a halogen atom, and R³ is a group of formula -NR⁴R⁵;
R⁴ is a C₁₋₆ alkyl group; and
R⁵ is a C₁₋₆ alkyl group; or
R⁴ and R⁵ together with the nitrogen atom to which they are bound form a heterocycloalkyl group comprising 5 or 6 ring atoms that are independently selected from C, N, S and O, which heterocycloalkyl group may optionally be substituted by 1, 2 or 3 substituents that are independently selected from a C₁₋₆ alkyl group, a C₁₋₆ alkoxy group, a hydroxy C₁₋₆ alkyl group and a hydroxy group;
or a salt, solvate or hydrate thereof.

Preferably, R¹ is an unsubstituted 2-pyridyl group, an unsubstituted 3-pyridyl group or an unsubstituted 4-pyridyl group.

Moreover preferably, R² is a fluorine atom and R³ is a group of formula -NR⁴R⁵.

Further preferably, R² is a group of formula -NR⁴R⁵ and R² is a fluorine atom.

Moreover preferably, the group of formula -NR⁴R⁵ is selected from the group consisting of morpholine, thiomorpholine, piperidine and pyrrolidine, all of which groups may optionally be substituted by 1, 2 or 3 substituents that are independently selected from a C₁₋₆ alkyl group, a C₁₋₆ alkoxy group, a hydroxyC₁₋₆ alkyl group.

Further preferably, R⁴ is a C₁₋₆ alkyl group and R⁵ is a C₁₋₆ alkyl group.

Moreover preferably, the group of formula -NR⁴R⁵ is selected from the group consisting of morpholine, thiomorpholine, piperidine, pyrrolidine, and diethylamino.

The most preferred compounds of the present invention are the compounds disclosed in the examples, or a salt thereof.

It is further preferred to combine the preferred embodiments of the present invention in any desired manner.

The expression "C₁₋₆ alkyl" refers to a saturated, straight-chain or branched or cyclic hydrocarbon group that contains from 1 to 6 carbon atoms, preferably from 1 to 4 carbon atoms, for example a methyl (Me, CH₃), ethyl (Et), *n*-propyl (nPr), isopropyl (iPr), *n*-butyl (nBu), isobutyl (iBu), *sec*-butyl (sBu), *tert*-butyl (tBu), *n*-pentyl, isopentyl or *n*-hexyl group.

The expression "C₁₋₆ alkoxy" refers to a group of formula -O-R wherein R is a C₁₋₆ alkyl group as defined above.

The expression "C₁₋₆ alkylthio" refers to a group of formula -S-R wherein R is a C₁₋₆ alkyl group as defined above.

The expression "hydroxy C₁₋₆ alkyl" refers to a C₁₋₆ alkyl group as defined above wherein one or more (preferably one) hydrogen atom is replaced by a hydroxyl group.

The expression "heterocycloalkyl" refers to a saturated or partially unsaturated cyclic group that contains one ring and 5 or 6 ring atoms that are independently selected from C, N, O and S with the proviso that at least one ring atom is selected from N, S and O. Preferably, at least one ring atom is a nitrogen atom. Examples are a morpholine, a thiomorpholine, a piperidine and a pyrrolidine group.

The term halogen refers to F, Cl, Br or I.

The term "optionally substituted" refers to a group which is unsubstituted or substituted by one or more (especially by one, two or three; preferably by one or two) substituents.

If a group comprises more than one substituent, these substituents are independently selected, i.e., they may be the same or different.

The therapeutic use of compounds according to formula (I), their salts, solvates and hydrates, respectively, as well as formulations and pharmaceutical compositions also lie within the scope of the present invention.

Compounds of formula (I) are capable of binding and covalently altering the structure of thioredoxin reductase TRxR1 thereby irreversibly inhibiting its function. It is to be understood, that in the present application, the human TRxR1 is particularly preferred, even though TRxR1 molecules of other origins (mouse) may also be contemplated.

The present invention also includes compounds of formula (I) for use in the treatment of diseases (especially human diseases) associated with an increased rate of cell growth (i.e., hyperproliferation), especially oncological diseases of various origins.

Although, quinoxaline 1,4-dioxide derivatives are known in the prior art [Hu Y. et al. Molecules, 2012, 77, 9683-9696; Das U. et al. Bioorg.Med. Chem., 2009, 17, 3909-3915, Cheng G. et al. Front. Pharmacol., 2016, 7, 1-21; Scherbakov A. M. et al. Cancer Invest., 2018, 3, 199-209; EP 3 138 565 A1; Zarranz B. et al. Arzneim.-Forsch./Drug Res., 2005, 55, 754-761; Balderas-Renteria et al., Current Medicinal Chemistry, 2012, 19, 4377-4398], it has been shown for the first time that derivatives of quinoxaline-2-carbonitrile 1,4-dioxide are capable of exerting of such effects by the inhibition of TrxR1. More specifically, the compounds of formula (I) according to the present invention are capable of providing said inhibition of TrxR1 by covalently modifying the enzyme by forming a covalent link between two oxidized Trp114 residues of two subunits from two separate TrxR1 dimers, as both found in cell extracts. Formation of covalently linked TrxR1 subunits became detectable in cells upon treatment with a compound of formula (I), in direct correlation with triggering of cell death that could be prevented by antioxidant treatment. Thus, even though antitumor inhibitors of TrxRl are disclosed in the literature [e.g. E.S.J.Amer, Adv Cancer Res 2017, 136:139-151, E. Chupakhin, M. Krasavin, Exp. Opin. Ther. Pat., 2021, 31 (8), 745-758 and cited refs], derivatives of quinoxaline-2-carbonitrile 1,4-dioxide that are capable to epy covalent modification of TrxR1 leading to its inhibition have not been identified prior to the present invention.

As mentioned above, it is known that derivatives of quinoxalin-2-carbonitrile 1,4-dioxide are capable to induce cell death in tumor cells, however, studies and application of these prior art derivatives as antitumor agents are hampered by their poor solubility in aqueous media [*PCT Int. Appl.* WO 2015167350, Zarranz B. et al. Bioorg. Med. Chem., 2004, 12, 3711-3721; Solano B. et al. J. Med. Chem., 2007, S0, 5485-5492; Das U. et al. Bioorg. Med. Chem., 2009, 17, 3909-3915]. In addition, a number of cytotoxic water-soluble derivatives of quinoxaline-2-carbonitrile 1,4-dioxides bearing diamine residues have been disclosed in RU2640304, however, the antitumor activity of this type of derivatives has not been confirmed by *in vivo* experimental data or clinical testing. In contrast, the compounds of formula (I) of the present invention, are capable to form water-soluble pharmaceutically acceptable salts with acids which strongly inhibit TrxRl and have significantly high antitumor activity, which has been confirmed by *in vivo* data. The compounds of formula (I) of the present invention show improved pharmaceutical properties and proven antitumor efficacy.

As mentioned above, the present invention inter alia relates to the application of compounds of formula (I) as inhibitors of the redox activity of TrxRl, which can be used for selective induction of ROS-associated death pathways in cancer cells of different origin (carcinomas of breast, lung, colon, liver, ovary and cervix, sarcoma, melanoma, leukemia, lymphoma, and neuroblastoma) including cells with resistance to other antitumor drugs. Any references to methods of treatment in the subsequent paragraphs of this description are to be interpreted as references to the compounds, pharmaceutical compositions and medicaments of the present invention for use in a method for treatment of the human (or animal) body by therapy (or for diagnosis). The present disclosure furthermore relates to a method of inhibiting the division of tumor cells by contacting the cells with an effective amount of a compound of formula (I) or a salt, solvate or hydrate thereof.

Moreover, the present invention relates to compounds of formula (I) for use in a method of medical treatment of tumor malignancies. In particular, the invention relates to compounds of formula (I) for use in the treatment of human tumors of different origin (carcinomas of breast, lung, colon, liver, ovary and cervix, sarcoma, melanoma, leukemia, lymphoma, neuroblastoma, etc.).

A method for treating a malignant neoplasm comprises the administration to a subject in need of treatment thereof of a therapeutically efficient amount of a compound of formula (I) or a salt, solvate or hydrate thereof, which is sufficient to inhibit TrxR in tumor cells.

The present invention further provides pharmaceutical compositions comprising one or more compounds of formula (I) or a salt, solvate or hydrate thereof as defined herein, optionally in combination with a pharmaceutically acceptable carrier and/or adjuvant.

The pharmaceutical compositions according to the present invention, and for use in accordance with the present invention, may comprise, in addition to one of the compounds of the present invention, a pharmaceutically acceptable excipient, buffer or stabilizer, or any other material well known to those skilled in the art and appropriate for the intended application. Such materials should be non-toxic and should not interfere with the efficacy of the active ingredient. Examples of techniques and protocols to this end may e.g., be found in Remington's Pharmaceutical Sciences, 16th edition, Osol, A. (ed.), 1980.

It is a further object of the present invention to provide a compound of formula (I) as defined herein or a pharmaceutical composition as defined herein for the preparation of a medicament for the treatment of tumors (especially human tumors).

Preferably the compounds of the present invention may be used for the treatment and/or prevention of various cancers including but not limited to carcinomas of breast, lung, colon, liver, ovary and cervix, sarcoma, melanoma, leukemia, lymphoma, and neuroblastoma.

According to one aspect, the present disclosure relates to a method for inhibiting thioredoxin reductase (TrxR1), comprising administering to a subject a compound of formula (I), or a salt, solvate or hydrate thereof, in an amount effective to inhibit TrxRl.

According to another aspect, the present invention relates to compounds of formula (I) for use in a method of inhibiting the division of tumor cells by providing for contact with cells of an effective amount of a compound of formula (I), or a salt, solvate or hydrate thereof. According to a preferred embodiment, the present invention relates to compounds of formula (I) for use in a method of inhibiting the division of tumor cells in which the malignant neoplasm is selected from a tumor of the carcinomas of breast, lung, colon, liver, ovary and cervix, sarcoma, melanoma, leukemia, lymphoma, or neuroblastoma. According to a further preferred embodiment, the present invention relates to compounds of formula (I) for use in a method of inhibiting the division of tumor cells in which the tumor cells have obtained drug resistance to other antitumor drugs.

According to yet another aspect, the present invention relates to compounds of formula (I) for use in a method for treating a malignant neoplasm, comprising administering to a subject in need of treatment thereof a compound of formula (I), or a salt, solvate or hydrate thereof, in an amount effective to inhibit TrxR.

A therapeutically effective amount of a compound in accordance with this invention means an amount of compound that is effective to prevent, alleviate or ameliorate symptoms of a disease or prolong the survival of the subject being treated. Determination of a therapeutically effective amount is within the skill in the art.

The therapeutically effective amount or dosage of a compound according to this invention can vary within wide limits and may be determined in a manner known in the art. Such dosage may be adjusted to the individual requirements in each particular case including the specific compound being administered, the route of administration, the condition being treated, as well as the patient being treated.

The salt of a compound of formula (I) is preferably a pharmacologically acceptable salt. Examples of pharmacologically acceptable salts of sufficiently basic compounds of formula (I) are salts of physiologically acceptable mineral acids like hydrochloric, hydrobromic, sulfuric and phosphoric acid; or salts of organic acids like methanesulfonic, p-toluenesulfonic, lactic, acetic, benzoic, glutamic, glycolic, propionic, tartaric, trifluoroacetic, citric, succinic, fumaric, maleic and salicylic acid.

Compounds of formula (I) may be solvated, especially hydrated. The hydratization/hydration may occur during the process of production or as a consequence of the hygroscopic nature of the initially water free compounds of formula (I). The solvates and/or hydrates may e.g. be present in solid or liquid form.

It should be appreciated that certain compounds of formula (I) may have tautomeric forms from which only one might be specifically mentioned or depicted in the following description. All these tautomeric forms and polymorphous forms are included in the invention.

As mentioned above, therapeutically useful agents that contain compounds of formula (I), their solvates, salts or formulations are also comprised in the scope of the present invention. In general, compounds of formula (I) will be administered by using the known and acceptable modes known in the art, either alone or in combination with any other therapeutic agent.

The compositions of the present invention may be prepared for any route of administration, e.g. oral, intravenous, cutaneous or subcutaneous, nasal, intramuscular or intraperitoneal. The precise nature of the carrier or other material will depend on the route of administration. For a parenteral administration, a parenterally acceptable aqueous solutions are employed, which is pyrogen free and has requisite pH, isotonicity and stability. Those skilled in the art are well able to prepare suitable solutions and numerous methods are described in the literature (for a brief review of methods of drug delivery, see Langer, Science 249:1 527-1533 (1990)). Preservatives, stabilizers, buffers, antioxidants and/or other additives may be included, as required. Dosage levels can be determined by those skilled in the art, taking into account the disorder to be treated, the condition of the individual patient, the site of delivery, the method of administration and other factors. Examples of the techniques and protocols mentioned above can be found in Remington's Pharmaceutical Sciences, 16th edition, Osol, A. (ed), 1980.

In general, in the case of oral or parenteral administration to adult humans weighing approximately 80 kg, a daily dosage of about 10 mg to about 10,000 mg, preferably from about 20 mg to about 1,000 mg, should be appropriate, although the upper limit may be exceeded when indicated. The daily dosage can be administered as a single dose or in divided doses, or for parenteral administration, it may be given as continuous infusion or subcutaneous injection.

According to a moreover preferred embodiment, the present invention provides compounds of formula (I) for use in a method for treating one or more diseases specified herein which comprises administering to a subject in need of such treatment a therapeutically effective amount of a compound of formula (I), or a salt, solvate or hydrate thereof.

According to a further preferred embodiment, the present invention provides compounds of formula (I) for use in a method for treating one or more diseases specified herein which comprises administering to a subject in need of such treatment a pharmaceutical composition comprising a compound of formula (I), or a salt, solvate or hydrate thereof.

The compounds of the present invention can be prepared according to a variety of general methods which are known in the art. The schemes described below only illustrate the possibility of synthesis of the claimed structures. Starting materials and reagents that are used for the preparation of compounds of formula (I) are either commercially available or they can be prepared by methods known in the state of the art. The reactions described herein are preferably carried out at atmospheric pressure and at a temperature of from about -78°C to 180°C, more preferably from 0°C to 120°C in an inert solvent or without solvent.

An inert solvent means a solvent that is inert under the conditions described in the text of the reaction, including, for example, benzene, toluene, acetonitrile, tetrahydrofuran, N,N-dimethylformamide, N,N-dimethylacetamide, N-methylpyrrolidone, dimethylsulfoxide, sulfolane, chloroform dichloromethane, dichloroethane, ethyl acetate, acetone, methyl ethyl ketone, methanol, ethanol, propanol, isopropanol, tert-butanol, dioxane, pyridine, etc.

A leaving group means a group whose name is usually associated with its use in synthetic organic chemistry, i.e., means an atom or group that is substituted under the reaction conditions. Examples of the leaving group include, but are not limited to, halogen, alkane or arenesulfonyloxy (such as methanesulfonyloxy, ethanesulfonyloxy, benzenesulfonyloxy, tosyloxy, and thienyloxy), alkoxy, alkylthio, hydroxy, and the like.

One of the methods for the preparation of the quinoxaline-2-carbonitrile 1,4-dioxides according to formula (I) is a method based on the substitution of leaving groups (LG) by treatment with the corresponded amine (HR²). In a preferred embodiment of the invention, this approach is efficient for the preparation of derivatives of 6-aminoquinoxaline-2-carbonitrile 1,4-dioxides 1 from quinoxaline-2-carbonitrile 1,4-dioxides of formula 2 as the LG in position 6 is activated for nucleophilic substitution by the residue of amines (R²) with the electron withdrawal effect of the cyano group located in position 2 (Scheme 1). If necessary, additional organic or inorganic bases (B) can be used to achieve a more complete conversion of the starting materials and/or a higher yield of the target products during the reaction.

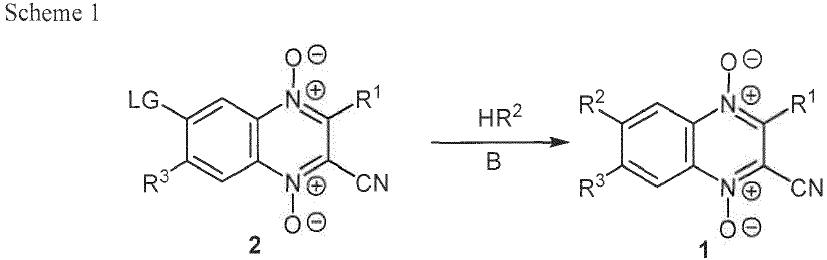

The starting quinoxaline-2-carbonitrile 1,4-dioxides **2** can be obtained by various methods, for example, as described in Scherbakov A.M. et al. Cancer Invest., 2018, 36, 199-209; Buravchenko G.I. et al. Bioorg. Chem., 2020, 104, 104324; Kotovskaya S.K. et al. Rus. J. Org. Chem., 2002, 38, 1046-1052; PCT Int. Appl. WO 2015167350, 2015**;** Hu Y. et al. Molecules, 2012, 17, 9683.

Another possible method of the preparation of compounds of formula (I) is based on the Beirut reaction of benzofuroxans with an amine residue (R³) and acylacetonitriles in the presence of bases B. According to a preferred embodiment of the invention, this approach is efficient for the synthesis of compounds of formula **1** with amino group (R²) in position 7 (Scheme 2), since in the Beirut reaction electron donating substituents in benzofuroxans **3** predominantly lead to the corresponded 7-substituted quinoxaline-2-carbonitrile 1,4-dioxides **1** [G.I. Buravchenko etal. Curr. Org. Synth., 2020, 17 (1), 29-39].

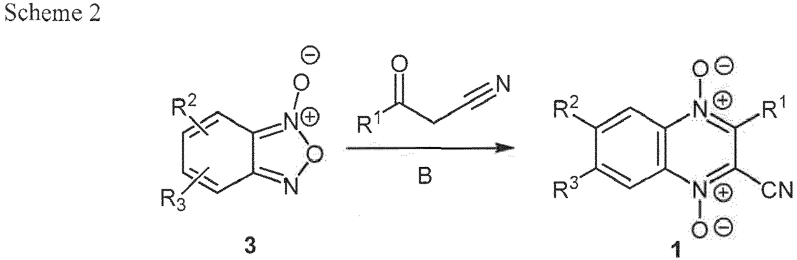

The starting benzofuroxans **3** can be obtained by various methods, for example, as described in Kotovskaya S.K. J. Fluorine Chem., 2004, 125, 421-428; Kotovskaya S.K. et al. Rus. J. Org. Chem., 2002, 38, 1046-1052.

### EXAMPLES

### A. Chemical syntheses

### Example 1

### 7-Fluoro-6-(morpholin-4-yl)-3-(pyridin-4-yl)quinoxaline-2-carbonitrile 1,4-dioxide (1a).

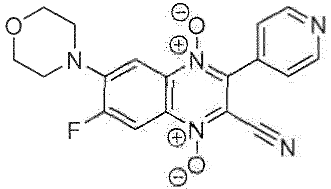

To a solution of 6,7-difluoro-3-(pyridine-4-yl)quinoxaline-2-carbonitrile 1,4-dioxide **2** (100 mg, 0.30 mmol, R¹= pyridine-4-yl, LG = R³ = F, Scheme 1) in *N*,*N*-dimethylformamide (5 ml) the morpholine (300 mg, 2.7 mmol) was added at r.t. The mixture was stirred for 2.5 h, poured into cold water (15 ml) and cooled. The precipitate was filtered, washed with water (30 ml) and dried. The crude precipitate was recrystallized from CH₂Cl₂-petroleum ether to provide the pure product. An orange powder, yield 82%, m.p. 230-233 °C. *Rf*=0.6 (CHCl₃-MeOH, 12:1). HPLC (LW = 260 nm, gradient B 20 / 70% (45 min)) t_{R} = 14.16 min, purity 96.9%. λ_{max.}, EtOH: 232, 284, 349, 407, 455 nm. ¹H NMR (400 MHz, DMSO- *d₆*), δ: 8.86 (2H, d, *J*= 4.9, C₆H₄N); 8.26 (1H, d, *J*= 13.0, H-8); 7.77 (1H, d, *J*= 8.2, H-5); 7.71 (2H, d, *J*= 4.9, C₆H₄N); 3.79 (4H, t, *J* = 6.2, CH₂O); 3.75 (4H, t, *J* = 6.2, CH₂N). HRMS (ESI) calculated for C₁₈H₁₅FN₅O₃⁺ [M+H]⁺ 368.1153, found 368.1146.

### 7-Fluoro-6-(morpholin-4-yl)-3-(pyridin-4-yl)quinoxaline-2-carbonitrile-1,4-dioxide hydrochloride (1a*HCl)

A mixture of compound **1a** (1 mmol), distilled water (5 ml) and hydrochloric acid (1.1 mmol) was heated until dissolved. The hot solution was filtered and evaporated to a minimum amount of water. Acetone (10 ml) and diethyl ether (10 ml) were added to the resulting solution for precipitating the product. The formed precipitate was filtered and washed with acetone-diethyl ether (2:1, 10 mL), diethyl ether (2×10 mL) and hexane (10 mL). An orange powder, yield 65%, m.p. 209-212 °C. HPLC (LW = 260 nm, gradient B 40 / 90% (29 min)) t_{R} = 14.24 min, purity 97.0%. λ_{max.}, EtOH: 232, 284, 349, 407, 453 nm. ¹H NMR (400 MHz, DMSO-*d₆*), δ: 9.06 (2H, d, *J*= 5.4, C₆H₄N); 8.28 (1H, d, *J*= 13.3, H-8); 8.11 (2H, d, *J*= 5.4, C₆H₄N); 7.76 (1H, d, *J*= 7.8, H-5); 6.75 (1H, br. s, NH⁺); 3.81-3.74 (8H, m, CH₂). HRMS (ESI) calculated for C₁₈H₁₅FN₅O₃⁺ [M+H]⁺ 368.1153, found 368.1171.

### 7-Fluoro-6-(morpholin-4-yl)-3-(pyridin-4-yl)quinoxaline-2-carbonitrile-1,4-dioxide methanesulfonate (1a*MsOH)

A mixture of compound **1a** (1 mmol), distilled water (5 ml) and methanesulphonic acid (1 mmol) was heated until dissolved. The hot solution was filtered and evaporated to a minimum amount of water. Acetone (10 ml) and diethyl ether (10 ml) were added to the resulting solution for precipitating the product. The formed precipitate was filtered and washed with acetone-diethyl ether (2:1, 10 mL), diethyl ether (2×10 mL) and hexane (10 mL). An orange powder, yield 57%, m.p. 190-193 °C. HPLC (LW = 260 nm, gradient B 20 / 70% (45 min)) t_{R} = 14.19 min, purity 95.9. λ_{max.}, EtOH: 232, 284, 349, 407, 455 nm. ¹H NMR (400 MHz, DMSO-*d₆*), δ: 9.04 (2H, d, *J*= 5.1, C₆H₄N); 8.25 (1H, d, *J*= 12.9, H-8); 8.10 (2H, d, *J*= 5.1, C₆H₄N); 7.74 (1H, d, *J*= 7.8, H-5); 5.76 (1H, br. s, NH⁺); 3.80-3.72 (8H, m, CH₂); 2.41 (3H, s, CH₃SO₃⁻). HRMS (ESI) calculated for C₁₈H₁₅FN₅O₃⁺ [M+H]⁺ 368.1153, found 368.1156.

### Example 2

### 6-Fluoro-7-(morpholin-4-yl)-3-(pyridin-4-yl)quinoxaline-2-carbonitrile-1,4-dioxide (1b).

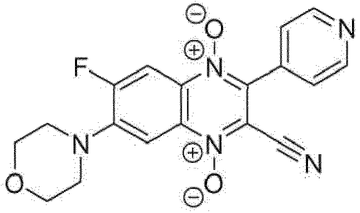

To a stirring mixture of the corresponding benzofuroxan **3** (1.0 mmol, R³ = morpholin-1-yl, R² = F, Scheme 2) and 3-oxo-3-(pyridin-4-yl)propanenitrile (4 mmol, R¹=pyridine-4-yl, Scheme 2) in acetone (5 mL), potassium carbonate (1.0 mmol) was added at room temperature. The mixture was stirred overnight. The solvent was removed under reduced pressure to give the crude product. The crude product was dissolved in water (100 mL) and the mixture was extracted with ethyl acetate (50 mL × 2). The combined organic layer was washed with water and dried over anhydrous Na₂SO₄. The solvent was removed under reduced pressure to give the precipitate, which was separated by column chromatography on silica gel in chloroform - ethyl acetate (5:1). Crystallization from hexane-dichlormethane (10:1) gave the pure product. An orange powder, yield 43%, m.p. 253-255 °C. *Rf*=0.5 (CHCl₃-MeOH, 12:1). HPLC (LW = 260 nm, gradient B 20 / 70% (45 min)) t_{R} = 13.82 min, purity 97.6%. λ_{max.}, EtOH: 221, 285, 376 nm. ¹H NMR (400 MHz, DMSO-*d₆*), δ: 8.87 (2H, d, *J*= 5.3, C₆H₄N); 8.27 (1H, d, *J*= 12.9, H-5); 7.79 (1H, d, *J*= 8.2, H-8); 7.72 (2H, d, *J*= 5.3, C₆H₄N); 3.81 (4H, t, *J*= 6.0, CH₂O); 3.39 (4H, t, *J*= 6.0, CH₂N). HRMS (ESI) calculated for C₁₈H₁₅FN₅O₃⁺ [M+H]⁺ 368.1153, found 368.1169.

### 6-Fluoro-7-(morpholin-4-yl)-3-(pyridin-4-yl)quinoxaline-2-carbonitrile-1,4-dioxide hydrochloride (1b*HCl)

This compound was obtained from **1b** similarly to **1a*HCl**, described in example 1. An orange powder, yield 71%, m.p. 220-223 °C. HPLC (LW = 260 nm, gradient B 20 / 70% (45 min)) t_{R} = 13.85 min, purity 91.9%. λ_{max.}, EtOH: 221, 285, 376 nm. ¹H NMR (400 MHz, DMSO-*d₆*), δ: 8.97 (2H, d, *J*= 5.3, C₆H₄N); 8.26 (1H, d, *J*= 13.3, H-5); 7.93 (2H, d, *J*= 5.3, C₆H₄N); 7.77 (1H, d, *J*= 7.8, H-8); 4.44 (1H, br.s, NH⁺); 3.78 (4H, t, *J*= = 6.2, CH₂O); 3.38 (4H, t, *J*= 6.2, CH₂N). HRMS (ESI) calculated for C₁₈H₁₅FN₅O₃⁺ [M+H]⁺ 368.1153, found 368.1153.

### 6-Fluoro-7-(morpholin-4-yl)-3-(pyridin-4-yl)quinoxaline-2-carbonitrile-1,4-dioxide methanesulfonate (1b*MsOH)

This compound was obtained from **1b** similarly to **1a*MsOH**, described in example 1. An orange powder, yield 64%, m.p. 197-200 °C. HPLC (LW = 260 nm, gradient B 20 / 70% (45 min)) t_{R} = 13.87 min, purity 95.2. λ_{max.}, EtOH: 221, 285, 376 nm. ¹H NMR (400 MHz, DMSO-*d₆*), δ: 8.94 (2H, d, *J*= 5.3, C₆H₄N); 8.26 (1H, d, *J*= 13.3, H-5); 7.77 (1H, d, *J*= 7.8, H-8); 7.88 (2H, d, *J*= 5.3, C₆H₄N); 4.10 (1H, br.s, NH⁺); 3.78 (4H, t, *J* = 6.0, CH₂O); 3.39 (4H, t, *J* = 6.0, CH₂N); 2.35 (3H, s, CH₃SO₃⁻). HRMS (ESI) calculated for C₁₈H₁₅FN₅O₃⁺ [M+H]⁺ 368.1153, found 368.1152.

### Example 3

### 7-Fluoro-6-(morpholin-4-yl)-3-(pyridin-3-yl)quinoxaline-2-carbonitrile-1,4-dioxide (1c).

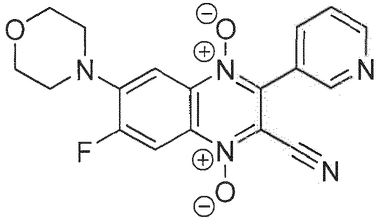

This compound was prepared from 6,7-difluoro-3-(pyridine-3-yl)quinoxaline-2-carbonitrile 1,4-dioxide **8** (R¹ = pyridine-3-yl, LG = R³ = F, Scheme 1) and morpholine according to procedure as described to compound **1a** (example 1). An orange powder, yield 36%, m.p. 207-210 °C. *Rf*=0.6 (CHCl₃-MeOH, 12:1). HPLC (LW = 260 nm, gradient B 20 / 70% (45 min)) t_{R} = 15.18 min, purity 96.6%. λ_{max.}, EtOH: 221, 291, 369, 443 nm. ¹H NMR (400 MHz, DMSO- *d₆*), δ: 8.90 (1H, br. s, C₆H₄N); 8.80 (1H, d, *J*= 4.7, H-5); 8.25 (1H, d, *J*= 12.9, H-8); 8.18 (1H, d, *J*= 7.8, C₆H₄N); 7.75 (1H, d, *J*= 7.8, C₆H₄N); 7.69-7.66 (1H, m, C₆H₄N); 3.82 (4H, br. m, CH₂O); 3.35 (4H, br. m, CH₂N). HRMS (ESI) calculated for C₁₈H₁₅FN₅O₃⁺ [M+H]⁺ 368.1153, found 368.1170.

### 7-Fluoro-6-(morpholin-4-yl)-3-(pyridin-3-yl)quinoxaline-2-carbonitrile-1,4-dioxide hydrochloride (1c*HCl)

This compound was obtained from **1c** similarly to **1a*HCl,** described in example 1. An orange powder, yield 63%, m.p. 196-199 °C. HPLC (LW = 260 nm, gradient B 20 / 70% (45 min)) t_{R} = 14.16 min, purity 96.9. λ_{max.}, EtOH: 230, 284, 348, 409, 455 nm. ¹H NMR (400 MHz, DMSO-*d₆*), δ: 8.99 (1H, s, C₆H₅N); 8.87 (1H, d, *J*= 6.2, C₆H₅N); 8.33 (1H, d, *J*= 7.8, C₆H₅N); 8.26 (1H, d, *J*= 12.9, H-5); 7.82 (1H, t, *J*= 7.8, C₆H₅N); 7.78 (1H, d, *J*= 7.9, H-8); 5.10 (1H, br.s, NH⁺); 3.78 (4H, t, *J*= 6.2, -CH₂O-); 3.37 (4H, t, *J*= 6.2, CH₂N); 2.40 (3H, s, CH₃SO₃⁻). HRMS (ESI) calculated for C₁₈H₁₅FN₅O₃⁺ [M+H]⁺ 368.1153, found 368.1177.

### 7-Fluoro-6-(morpholin-4-yl)-3-(pyridin-3-yl)quinoxaline-2-carbonitrile-1,4-dioxide methanesulfonate (1c*MsOH)

This compound was obtained from **1c** similarly to **1a*MsOH,** described in example 1. An orange powder, yield 38%, m.p. 204-207 °C. HPLC (LW = 260 nm, gradient B 20 / 70% (45 min)) t_{R} = 15.18 min, purity 85.8. λ_{max.}, EtOH: 233, 291, 349, 414, 453 nm. ¹H NMR (400 MHz, DMSO-*d₆*), δ: 8.99 (1H, s, C₆H₄N); 8.87 (1H, d, *J*= 6.2, C₆H₄N); 8.33 (1H, d, *J*= 7.8, C₆H₄N); 8.26 (1H, d, *J*= 12.9, H-5); 7.82 (1H, t, *J*= 7.8, C₆H₄N); 7.78 (1H, d, *J*= 7.9, H-8); 5.10 (1H, br.s, NH⁺); 3.78 (4H, t, *J* = 6.2, CH₂O); 3.37 (4H, t, *J* = 6.2, CH₂N); 2.40 (3H, s, CH₃SO₃⁻). HRMS (ESI) calculated for C₁₈H₁₅FN₅O₃⁺ [M+H]⁺ 368.1153, found 368.1189.

### Example 4

### 6-Fluoro-7-(morpholin-4-yl)-3-(pyridin-3-yl)quinoxaline-2-carbonitrile-1,4-dioxide (1d).

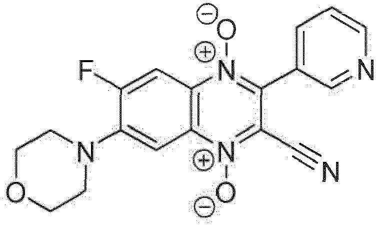

This compound was prepared from 6-fluoro-5-(morpholin-4-yl)benzofuroxan **(3,** R³ = morpholin-1-yl, R² = F, Scheme 2) and 3-oxo-3-(pyridin-3yl)propanenitrile (R¹=pyridine-3-yl, Scheme B,) according to procedure described for compound **1b** (example 2). An orange powder, yield 35%, m.p. 241-243 °C. *Rf*=0.5 (CHCl₃-MeOH, 12:1). HPLC (LW = 260 nm, gradient B 20 / 70% (45 min)) t_{R} = 15.09 min, purity 99.1%. λ_{max.}, EtOH: 221, 290, 372, 445 nm. ¹H NMR (400 MHz, DMSO- *d₆*), δ: 8.89 (1H, s, C₆H₄N); 8.78 (1H, d, *J*= 5.1, C₆H₄N); 8.26 (1H, d, *J*= 13.8, H-5); 8.17 (1H, d, *J*= 8.0, C₆H₄N); 7.75 (1H, d, *J*= 8.1, H-8); 7.67 (1H, t, *J* = 8.0, C₆H₄N); 3.80 (4H, t, *J* = 6.0, CH₂O); 3.36 (4H, t, *J* = 6.0, CH₂N). HRMS (ESI) calculated for C₁₈H₁₅FN₅O₃⁺ [M+H]⁺ 368.1153, found 368.1178.

### 6-Fluoro-7-(morpholin-4-yl)-3-(pyridin-3-yl)quinoxaline-2-carbonitrile-1,4-dioxide hydrochloride (1d*HCl)

This compound was obtained from **1d** similarly to compound **1a*HCl**, described in example 1. An orange powder, yield 71%, m.p. 208-211 °C. HPLC (LW = 260 nm, gradient B 40 / 90% (29 min)) t_{R} = 14.24 min, purity 97.0%. λ_{max.}, EtOH: 221, 290, 370 nm. ¹H NMR (400 MHz, DMSO-*d₆*), δ: 8.95 (1H, s, C₆H₄N); 8.83 (1H, d, *J*= 5.1, C₆H₄N); 8.28 (1H, dd, *J³*= 7.8, *J⁴*= 5.0, C₆H₄N); 8.25 (1H, d, *J*= 13.3, H-5); 7.76 (1H, d, *J*= 7.8, C₆H₄N); 7.75 (1H, d, *J*= 7.9, H-8); 4.49 (1H, br. s, NH⁺); 3.80 (4H, t, *J* = 6.3, CH₂O); 3.34 (4H, t, *J* = 6.3, CH₂N). HRMS (ESI) calculated for C₁₈H₁₅FN₅O₃⁺ [M+H]⁺ 368.1153, found 368.1174.

### Example 5

### 6-Fluoro-7-(morpholin-4-yl)-3-(pyridin-2-yl)quinoxaline-2-carbonitrile-1,4-dioxide (1e).

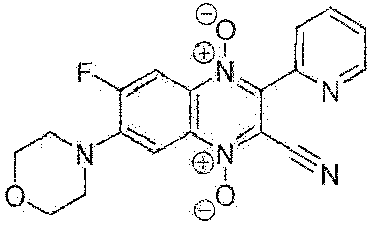

This compound was prepared from 6-fluoro-5-(morpholin-4-yl)benzofuroxan **(3,** Y R³ = morpholin-1-yl, R² = F, Scheme 2) and 3-oxo-3-(pyridin-2yl)propanenitrile (R¹ = pyridine-2-yl, Scheme B) according to procedure described for compound **1b** (example 2). An orange powder, yield 35%, m.p. 241-243 °C. *Rf*=0.43 (CHCl₃-MeOH, 12:1). HPLC (LW = 260 nm, gradient B 40 / 60% (25 min)) t_{R} = 8.8 min, purity 97.6%. λ_{max.}, EtOH: 220, 283, 370, 423 nm. ¹H NMR (400 MHz, DMSO- *d₆*), δ: 8.83 (1H, d, C₆H₄N); 8.28 (1H, d, *J*= 13.4, H-5); 8.12 (2H, m, C₆H₄N); 7.74 (1H, d, *J*= 8.1, H-8); 7.64 (1H, m, C₆H₄N); 3.80 (4H, t, *J*= 6.0, CH₂O); 3.34 (4H, t, *J* = 6.0, CH₂N). HRMS (ESI) calculated for C₁₈H₁₅FN₅O₃⁺ [M+H]⁺ 368.1153, found 368.1123.

### Example 6

### 6-Fluoro-7-(pyrrolidin-1-yl)-3-(pyridin-3-yl)quinoxaline-2-carbonitrile-1,4-dioxide (1f).

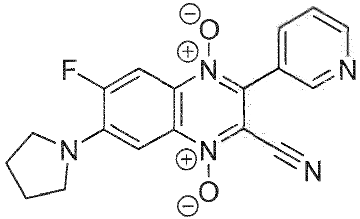

This compound was obtained from 6-fluoro-5-(pyrrolidin-1-yl)-benzofuroxan (**3**, R³ = pyrrolidin-1-yl, R² = F, Scheme 2) and 3-oxo-3-(pyridin-3-yl)propanenitrile (R¹ = pyridine-3-yl, Scheme B) according to the procedure described for compound **1b** (example 2). An orange powder, yield 31%, m.p. 215-217 °C. HPLC (LW = 260 nm, gradient B 20 / 70% (45 min)) t_{R} = 19.91 min, purity 98.8%. λ_{max.}, EtOH: 228, 298, 323, 372, 509 nm. ¹H NMR (400 MHz, DMSO-*d₆*), δ: 8.90 (1H, s, C₆H₄N); 8.77 (1H, d, *J*= 5.3, C₆H₄N); 8.18 (1H, d, *J*= 8.0, C₆H₄N); 8.11 (1H, d, *J*= 13.7, H-5); 7.66 (1H, dd, *J³*= 8.0, *J⁴*= 5.3, C₆H₄N); 3.62 (4H, br. s, CH₂N); 2.00 (4H, br. s, CH₂). HRMS (ESI) calculated for C₁₈H₁₅FN₅O₂⁺ [M+H]⁺ 352.1204, found 352.1233.

### Example 7

6-Fluoro=7-(piprtidine-1-yl)-3-(pyridin-3-yl)quinoxaline-2-carbonitrile-1,4-dioxide (**1g**)

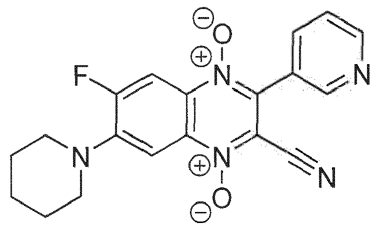

This compound was prepared from 6-fluoro-5-(piperidin-4-yl)benzofuroxan (**3**, R³ = pyrrolidin-1-yl, R² = F, Scheme 2) and 3-oxo-3-(pyridin-3-yl)propanenitrile (R¹ =pyridine-3-yl, Scheme B) according to the procedure described for **1b** (example 2). An orange powder, yield 52%, m.p. 228-229 °C. HPLC (LW = 290 nm, gradient B 40 / 95% (45 min)) t_{R} = 14.79 min, purity 98.9%. λ_{max.}, EtOH: 224, 291, 377, 460 nm. ¹H NMR (400 MHz, DMSO-*d₆*), δ: 8.89 (1H, s, C₆H₄N); 8.87 (1H, d, *J*= 4.9, C₆H₄N); 8.21 (1H, d, *J*= 13.1, H-5); 8.17 (1H, d, *J*= 8.0, C₆H₄N); 7.72 (1H, d, *J*= 8.2, H-8); 7.66 (1H, t, *J* = 8.0, C₆H₄N); 3.33 (4H, t, *J* = 6.2, CH₂N); 1.71 (4H, m, CH₂); 1.64 (2H, m, -CH₂-). HRMS (ESI) calculated for C₁₉H₁₇FN₅O₂⁺ [M+H]⁺ 366.1361, found 366.1323.

### Example 8

### 7-Fluoro-3-(pyridin-4-yl)-6-thiomorpholinoquinoxaline 1,4-dioxide (1h)

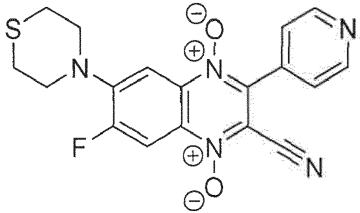

This compound was prepared from 6,7-difluoro-3-(pyridine-4-yl)quinoxaline-2-carbonitrile 1,4-dioxide **2** (R¹ = pyridine-4-yl, LG = R³ = F, Scheme 1) and thiomorpholine according to the procedure as described for compound **1a** (example 1). An orange powder, yield 54%, m.p. >251 °C. *Rf*=0.53 (CHCl₃-MeOH, 10:1). HPLC (LW = 260 nm, gradient B 15 / 75% (45 min)) t_{R} = 15.51 min, purity 95.5%. λ_{max.}, EtOH: 235, 284, 339, 410, 458 nm. ¹H NMR (400 MHz, DMSO- *d₆*), δ: 8.80 (2H, d, *J*= 5.0, C₆H₄N); 8.26 (1H, d, *J*= 13.1, H-8); 7.75 (1H, d, *J*= 8.1, H-5); 7.63 (2H, d, *J*= 5.0, C₆H₄N); 3.71 (4H, s, CH₂N). 2.79 (2H, s, CH₂S). HRMS (ESI) calculated for C₁₈H₁₅FN₅O₂S⁺ [M+H]⁺ 384.0925, found 384.0911.

### Example 9

### 6-(Diethylamino)-7-fluoro-3-(pyridin-4-yl)quinoxaline-2-carbonitrile 1,4-dioxide (1i)

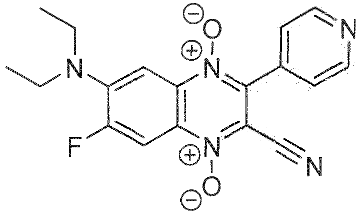

This compound was prepared from 6,7-difluoro-3-(pyridine-4-yl)quinoxaline-2-carbonitrile 1,4-dioxide **2** (R¹ = pyridine-4-yl, LG = R³ = F, Scheme 1) and diethylamine according to the procedure as described for compound **1a** (example 1). An orange powder, yield 59%, m.p. 212-212 °C. *Rf*=0.63 (CHCl₃-MeOH, 12:1). HPLC (LW = 260 nm, gradient B 15 / 75% (45 min)) t_{R} = 13.21 min, purity 95.1%. λ_{max.}, EtOH: 230, 276, 333, 411, 450 nm. ¹H NMR (400 MHz, DMSO- *d₆*), δ: 8.81 (2H, d, *J*= 4.9, C₆H₄N); 8.22 (1H, d, *J*= 13.0, H-8); 7.71 (1H, d, *J*= 8.1, H-5); 7.65 (2H, d, *J*= 4.9, C₆H₄N); 3.53 (4H, q, CH₂N). 1.34 (6H, t, CH₃). HRMS (ESI) calculated for C₁₈H₁₇FN₅O₂⁺ [M+H]⁺ 354.1361, found 354.1378.

### B. Biological evaluation

### Materials and Methods

### Cell culture and hypoxia treatment

Breast, colon, ovarian, cervical, liver and lung carcinoma cell lines were grown in DMEM supplemented with 10% FBS, and antibiotics were cultured at 37 °C and 5% CO₂. Osteosarcoma, melanoma, lymphoma, neuroblastoma and leukemia cell lines were cultured in RPMI 1640 with 10% FBS and antibiotics. An oxygen concentration of 0.3% was obtained by flushing a sealed box containing the cells with premixed gas composed of 95% nitrogen (N₂) and 5% CO₂ (AGA AB, Sweden). To obtain an oxygen concentration of 0.05%, the cells were flushed with the premixed gas continuously throughout the experiment.

### Growth suppression assay

Cells were grown in 96-well plates at a density of 3000 cells per well with or without doxycycline and treated with compounds. After 48 hours of incubation the proliferative cell reagent resazurin (Thermo Fisher Scientific) was added to the cells. The degree of resazurin reduction, which reflects cell viability, was measured by microplate reader by monitoring the absorbance at 570 nm and 600 nm, according to the manufacturer.

### Apoptosis detection by FACS

Cells were placed on 12-well plate at a density of 30000/cm² and treated with compounds. After 24h incubation cells were harvested by trypsinization, fixed with 70% ethanol, treated with RNase A (0.25 mg/ml) and stained with Annexin V according to manufacturer's instructions (Thermo Fisher Scientific). Samples were analyzed on a Becton Dickinson FACScan. Data were analyzed by the CellQuest software, version 3.2.1.

### Oxidative stress measurement

Cells were treated with compounds and incubated 30 min with 10 µM DCF-DA in serum free medium. Afterward, cells were trypsinized, washed with PBS and fluorescence was analyzed by a FACSCalibur flow cytometer (BD Biosciences) using FL1-H channel.

### Western blot analysis

The extraction of total cell lysates and western blot were performed according to standard procedure. The antibodies used for immunoblotting were: p53 (sc-126, Santa Cruz Biotechnology), γH2AX (#07-164, Millipore), TrxRl 19A1 (AbFrontier). Anti-β-actin monoclonal antibody (Millipore) was used as loading control. The horseradish peroxidase-coupled secondary antibodies (Jackson ImmunoResearch) and SuperSignal^{™} West Dura Extended Duration Substrate detection system (Thermo Fischer Scientific), and images were visualized using ChemiDoc Imaging System (Bio-Rad).

### Detection of TrxR dimers

MCF7 or HCT116 cells were treated in culture with 5 or 10 µM of 1d*HCl (Example 4) in complete medium for 2h or 6h. For each case, duplicate drug treated samples were produced plus duplicate control treatment samples using equal volumes of the drug vehicle dimethyl sulfoxide. Detached cells from each biological replicate were washed, suspended in PBS containing protease inhibitors and cells lysed through freeze thaw cycles in liquid N₂, according to the reference protocol. Soluble fraction was separated from precipitated protein by ultracentrifugation at 100000 g for 30 minutes at 4°C and TrxR1 dimer formation was analysed using Western blot as described above.

### TrxRl enzymatic activity measurement in vitro

TrxR1 enzyme was recombinantly produced with high Sec contents in a specific Release Factor 1-depleted E. coli strain and purified as previously described (Arnér ESJ, Holmgren A, in Current Protocols in Toxicology, Maines M, Costa L, Reed D, Sassa S, Eds. (John Wiley & Sons, Inc., New York, 2000), pp. 7.4.1-7.4.14). TrxRl and GSR (Sigma) recombinant enzyme assays were performed in a reaction buffer containing 50 mM Tris pH 7.5 with 2 mM EDTA and 0.1 mg/mL bovine serum albumin as described previously (Arnér ESJ, Holmgren A, in Current Protocols in Toxicology, Maines M, Costa L, Reed D, Sassa S, Eds. (John Wiley & Sons, Inc., New York, 2000), pp. 7.4.1-7.4.14). Briefly, assays were performed in triplicate using 96-well plates, with activity inferred from absorbance changes using a Spectra Max plate reader. Results of recombinant enzyme assays were normalized to DMSO and to no enzyme controls.

### TrxRl enzymatic activity measurement in cellulo

MCF7 cells were treated with 5 or 10 µM 1d*HCl or DMSO as a control. Cells were collected 2 h and 6 h after treatment, washed twice with PBS, and lysed in 100 µL of lysis buffer (50 mM Tris-HCl, 2mM EDTA, 0.15 M NaCl, 1% Triton X-100, protease inhibitors (Roche), pH 7.5). Cells were scraped, collected and snap frozen. After three freeze-thawing cycles the lysate was spinned down, supernatant collected and protein concentration measured using Pierce bicinchoninic acid assay kit (BCA, from Thermofisher Scientific) according to the manufacturer's protocol. To measure TrxR activity, the end-point TXN-dependent insulin reduction assay was used, essentially as described previously (WC Stafford, et al. Irreversible inhibition of cytosolic thioredoxin reductase 1 as a mechanistic basis for anti-cancer therapy, Sci Transl Med (2018), Feb 14;10(428):eaaf7444. doi: 10.1126/scitranslmed.aaf7444). In short, 7-5-9 µg of total protein from the cell lysate was incubated with 0.16 µM human insluin (Sigma), 0.33 mM NAPDH (Saveen Werner) and 16 µM human TXN recombinantly produced as previously described (WC Stafford, et al, Irreversible inhibition of cytosolic thioredoxin reductase 1 as a mechanistic basis for anti-cancer therapy, Sci Transl Med (2018), Feb 14;10(428):eaaf7444. doi: 10.1126/scitranslmed.aaf7444) in TE buffer (50 mM Tris-HCl, 2mM EDTA, pH 7.5) in a total volume of 50 µL and was then incubated at 37°C. After 0, 15 and 30 min, 10 µL aliquots were taken and combined with 6 M guanidine-HCl and 2.5 mM DTNB in 96-well plates (PerkinElmer), whereupon the absorbance at 412 nm was measured using microplate spectrophotometer (TECAN). Levels of active TrxR were normalised to DMSO control samples from each treatment timepoint.

Glutathione reductase inhibition assay was performed according to (S. Busker, W. Qian, M. Haraldsson, B. Espinosa, L. Johansson, S. Attarha, 1. Kolosenko, J. Liu, M. Dagnell, D. Grandér, E.S.J. Arnér, K. PokrovskajaTamm, B.D.G. Page. Irreversible TrxRl inhibitors block STAT3 activity and induce cancer cell death, Sci. Adv., 6 (2020), 10.1126/sciadv.aax7945). 5 or 10 µM compound was incubated with 175 µM GSH in TE buffer. At 0, 1, 2, 4, 6 and 24 h 10 µL of the reaction was mixed with 100 µL 1 mM DTNB, in order to assess the concentration of reduced GSH remaining in the reaction.

### Statistical analysis

For all experiments, statistical analyses were performed using GraphPad Prism 5. Results are given as mean ± s.d. To evaluate statistical significance, Student's t-test (unpaired) or one-way ANOVA with Bonferroni's multiple comparison test were performed. P-values ≤ 0.05 were considered statistically significant.

### In vivo experiments

Animal experiments were approved by the regional ethics committee for animal research (N391/11 and N26/11), appointed and under the control of the Swedish Board of Agriculture and the Swedish Court. The animal experiments were in accordance with national regulations (SFS 1988:534, SFS 1988:539 and SFS 1988:541).

For the B16 melanoma mouse model, 6-weeks-old female C57BL/6 mice were injected subcutaneously with 1x106 B16 cells. When the tumors reached 80-120 mm³, 10 mg/kg of **1d*HCl** were administered intraperitoneally every other day for 9 days (days 1, 3, 5, 7, 9). Tumor volume was measured by caliper three times per week, and the volume was calculated based on the formula (in ml): L/10*W/10*W/10*0.44 (L=length, W=width of tumor). Mice were sacrificed when tumors reached 2 cm³ or upon bleeding.

### Results and discussion

### Growth suppression of tumor cells

Two carcinoma cell lines, MCF7 (breast carcinoma) and HCT116 (colon carcinoma) grown in 96-well plates, were treated with increasing concentrations of the synthesized compounds, ranging from 0.4 to 25.6 M. After 48 hours of incubation the proliferative cell reagent resazurin was added to the cells. The degree of resazurin reduction, which reflects cell viability, was measured by microplate reader by monitoring the absorbance at 570 nm and 600 nm, according to the manufacturer and the IC₅₀ was determined (Table 1). Since water-soluble **1d*HCl** had superior IC₅₀ in both HCT116 and MCF7 cells, this compound was selected for further testing.

**Table 1:**

| **Compound** | **Solubility** | **IC₅₀ value MCF7-Cas9** | **IC₅₀ value HCT116-Cas9** |
|---|---|---|---|
| **1b** | Warm DMSO | 4.1 ± 0.X µM | 2.8 ± 0.X µM |
| **1d** | Warm DMSO | 4.1 ± 0.X µM | 4.1 ± 0.X µM |
| **1g** | Warm DMSO | 4.1 ± 0.X µM | 2.8 ± 0.X µM |
| **1b*HCl** | H₂O | 3.8 ± 0.X µM | 3.2 ± 0.X µM |
| **1b*MsOH** | H₂O | 4.2 ± 0.X µM | 3.4 ± 0.X µM |
| **1c*HCl** | H₂O | >10 | 10.9 ± 0.X µM |
| **1a*HCl** | H₂O | >10 | 9.3 ± 0.X µM |
| **1a*MsOH** | H₂O | 2.4 ± 0.X µM | 1.2 ± 0.X µM |
| **1d*HCl** | H₂O | 1.6 ± 0.X µM | 0.69 ± 0.X µM |
| **1f** | Warm DMSO | 3.8 ± 0.X µM | 2.2 ± 0.X µM |
| **1a** | Warm DMSO | 6.9 ± 0.X µM | 3.5 ± 0.X µM |
| **1c** | Warm DMSO | - | 3.3 ± 0.X µM |

### Growth suppression by the soluble compound 1d*HCl in a panel of 27 cancer cell lines.

The panel of cancer cell lines, including carcinomas of breast (MDA-MB231, SKBR3, T47D, MCF7; BT549, BT20, MDA-MB468), colon, (SW480, RKO, HT29, HCT116) ovarian (OVCAR3, OVCAR4, OVCAR8), cervical (SW756, C33A), liver (HEPG2) and lung (H23), osteosarcoma (SJSA and U2OS), melanoma (ME180), lymphoma (Raji, HDLM2), neuroblastoma (SKNAS, IMR32) and leukemia (NB4, K562) were treated with increasing concentrations of **1d*HCl:** 0.4; 0.8; 1.6; 3.2; 6.4; 12.8; 25.6 µM in 96-well plates. After 48 hours of incubation the proliferative cell reagent resazurin was added to the cells. The degree of resazurin reduction, which reflects cell viability, was measured by microplate reader by monitoring the absorbance at 570 nm and 600 nm, according to the manufacturer, and the IC₅₀ and AUC (area under curve) were determined (Table 2). As could be seen in Table 2, **1d*HCl** suppressed the growth of cancer cell lines of a different origin, including carcinomas, osteosarcomas, melanoma, lymphoma, leukemia and neuroblastoma. These findings open up a possibility of the application of the present invention in anti-cancer therapy in a broad range of tumor types.

### Table 2:

a) Breast cancer

| **1d*HCl (µM)** | **MDA- MB231** | **SKBR3** | **T47D** | **MCF7** | **BT549** | **BT20** | **MDA-MB-468** |
|---|---|---|---|---|---|---|---|
| **IC₅₀ (µM)** | **0,78** | **2,13** | **2,74** | **2,54** | **1,70** | **5,91** | **2,78** |
| **AUC** | **1,21** | **3,06** | **3,93** | **3,64** | **2,52** | **9,64** | **4,04** |

b) Colon

| **1d*HCl (µM)** | **HCT116** | **RKO** | **HT29** | **SW480** |
|---|---|---|---|---|
| **IC₅₀ (µM)** | **0,86** | **0,538** | **3,50** | **3,72** |
| **AUC** | **1,37** | **0,942** | **4,77** | **4,78** |

c) Ovarian

| **1d*HCl (µM)** | **OVCAR3** | **OVCAR4** | **OVCAR8** |
|---|---|---|---|
| **IC₅₀ (µM)** | **2,64** | **1,57** | **2,36** |
| **AUC** | **4,813** | **3,7** | **2,98** |

d) Cervical

| **1d*HCl (µM)** | **SW756** | **ME180** | **C33A** |
|---|---|---|---|
| **IC50 (µM)** | **1,54** | **2,23** | **variable** |
| **AUC** | **2,84** | **2,72** | **21** |

e) Lymphomas and Leukemias

| **1d*HCl (µM)** | **Raji** | **HDLM2** | **NB4** | **K562** |
|---|---|---|---|---|
| **IC₅₀ (µM)** | **1,22** | **1,27** | **variable** | **2,95** |
| **AUC** | **2,93** | **4,68** | **25,4** | **4,39** |

f) Sarcomas, Neuroblastoma. Liver and Lung

| | | | | | | |
|---|---|---|---|---|---|---|
| **1d*HCl (µM)** | **SJSA** | **U2O5** | **SKNAS** | **IMR32** | **HepG2** | **H23** |

| **IC₅₀ (µM)** | **4,08** | **1,04** | **4,68** | **5,15** | **3,59** | **3,54** |
|---|---|---|---|---|---|---|
| **AUC** | **4,96** | **2,10** | **7,73** | **6,40** | **4,54** | **5,38** |

### 1d*HCl induces cell death by apoptosis in cancer cells

To find out whether the growth suppression by 1d*HCl is due to triggering cell death by apoptosis, we performed the Annexin V-mediated staining of phosphatidylserine, exposed on surface of cells undergoing apoptotic cell death. We detected Annexin V-positive cells using FACS. Thereby, it has been found that 24h treatment with 2 µM **1d*HCl** of MCF7 cells induced Annexin V-positive cells statistically significantly already 24h after treatment.

### 1d*HCl induces oxidative stress in cancer cells

Cancer cells are particularly vulnerable to oxidative stress as a result of TrxR1 inhibition because of inherently increased oxidative stress because of oncogenic signaling, distorted metabolism, impaired mitochondrial function, and other mechanisms. To get insight into the mechanism of the anti-tumor activity of **1d*HCl,** we tested the level of oxidative stress upon treatment. We measured the induction of reactive oxygen species (ROS), using 2'-7'-Dichlorodihydrofluorescein diacetate (DCFH-DA)-based assay. We found a significant increase in DCF fluorescence already 2h after treatment with **1d*HCl.** After 8h of treatment, the induction of reactive oxygen species (ROS) was much higher.

### Induction of oxidative stress is the mechanism of growth suppression by 1d*HCl

To find out whether oxidative stress plays a role in cancer cell death induced by **1d*HCl,** we tested the extent of growth suppression by the treatment in the presence of antioxidant N-acetyl cysteine (NAC), which neutralize ROS. We pre-treated HCT116 cells with 5 mM NAC. After 16h of incubation with NAC, we treated HCT116 cells with 2.5, 5, and 10 µM of **1d*HCl**. We measured the growth suppression 24h after treatment. Thereby, it was found that prevention of ROS accumulation by NAC almost completely abolished the growth suppression by **1d*HCl**. Thus, we conclude that the mechanism of cancer cell growth suppression is dependent on the induction of oxidative stress by the compounds of the present invention.

### Cell death induced by 1d*HCl is p53-independent

Since p53 is a major inducer of apoptosis, including ROS-induced apoptosis, we tested whether p53 plays a role in growth suppression by **1d*HCl**. We used wild type p53 HCT116 cells and their isogenic p53-negative cell line HCT116 p53-/-, as well as a pair of wild type p53 A375 and p53-negative A375p53-/- melanoma cells, generated using CRISPR-Cas9, as described previously [Zhou X, Singh M, Sanz Santos G, Guerlavais V, Carvajal LA, Aivado M, Zhan Y, Oliveira MMS, Westerberg LS, Annis DA, Johnsen JI, Selivanova G. Pharmacologic activation of p53 triggers viral mimicry response thereby abolishing tumor immune evasion and promoting anti-tumor immunity Cancer Discov. (20121), 11(12), 3090-3105]. It was found that the growth suppression by **1d*HCl** was not affected in the absence of p53. This allowed us to conclude that the tumor suppression is p53 -independent. These data are in line with our cancer cell line screen (Table 2). Mutant p53 cancer lines, which are sensitive to growth suppression by **1d*HCl,** include carcinomas of breast MDA-MB231, SKBR3, T47D, BT549, and MDA-MB468, colon SW480 and HT29, ovarian OVCAR3, OVCAR4, and OVCAR8, cervical SW756 and C33A, and lung H23, and lymphoma Raji. Thus, **1d*HCl** could be used to treat p53-mutant tumors, which are most aggressive and metastatic. The p53-induced apoptosis is essential for the efficiency of the majority of DNA-damaging chemotherapeutic agents, which are often not efficient in p53-mutant tumors. Since mutations in p53 occur in 50% of all tumors, our finding that the growth suppression by **1d*HCl** is not mediated by p53 opens up a possibility for a very broad application of **1d*HCl-**based anti-cancer therapy.

### 1d*HCl selectively inhibits the enzymatic activity of recombinant purified TrxRl in vitro

It has been shown that the selenoprotein thioredoxin reductase 1 (TrxRl) and glutaredoxin (GR) are crucial enzymes in cellular redox systems. TrxRl reduces of the active site of thioredoxin 1 (Trx1) using NADPH, whereas GR reduces glutathione, thereby these enzymes are required for the many cell functions, including the promotion of cell viability and proliferation (Arner ESJ: Targeting the Selenoprotein Thioredoxin Reductase 1 for Anticancer Therapy. Adv Cancer Res 2017, 136:139-151). Over the last decades, it was shown that the inhibition of the cytosolic selenoprotein TrxRl (TXNRD1) as a central regulator of the Trx system, may be pharmacologically inhibited to achieve selective cancer cell killing [Gencheva R, Arnér ESJ.Annu Rev Pharmacol Toxicol. 2021 Aug 24. doi: 10.1146/annurev-pharmtox-052220-102509]. This idea is based on a number of observations that cancer cells are uniquely vulnerable to TrxR inhibition by virtue of their increased reliance on detoxifying systems counteracting the ROS overproduction accompanying a cancer-specific deregulated metabolism and increased mitogenic signaling.

Since we found that **1d*HCl** is a strong inducer of oxidative stress, we set out to test whether **1d*HCl** can inhibit thioredoxin reductase or glutaredoxin. We used recombinant purified TrxRl and GR in an in vitro assay as described in methods section. It has been found that **1d*HCl** efficiently inhibited the enzymatic activity of TrxR1 in a dose- and time-dependent manner. In contrast, **1d*HCl** did not inhibit GR activity. Thus, we conclude that **1d*HCl** selectively inhibits the enzymatic activity of purified TRxR1, but not GR.

### 1d*HCl selectively inhibits the enzymatic activity of TrxR1 in cellulo

Having established that **1d*HCl** inhibits TrxRl activity *in vitro,* it was important to confirm that **1d*HCl** targets TrxRl in living cells. Therefore, we tested whether **1d*HCl** is able to inhibit the enzymatic activity of TrxRl in MCF7 breast cancer cells. Our results demonstrated that **1d*HCl** inhibited TrxRl activity in a dose- and time-dependent manner: **1d*HCl** inhibited TrxR1 activity in cells by 70-80%, depending on concentration, already 2h after treatment. In contrast, GR activity was not inhibited by **1d*HCl** in cells, which is in line with the *in vitro* results. Thus, we conclude that **1d*HCl** selectively targets TrxRl in cancer cells and inhibits its activity, leading to the induction of ROS and oxidative stress.

### 1d*HCl inhibits TrxR1 by inducing covalent dimer formation

Our previous studies identified a previously unknown mechanism of inhibition of TrxRl by the p53-reactivating compound RITA [Xu J, Eriksson SE, Cebula M, Sandalova T, Hedström E, Pader I, Cheng Q, Myers CR, Antholine WE, Nagy P, Hellman U, Selivanova G, Lindqvist Y, Arnér ES. The conserved Trp114 residue of thioredoxin reductase 1 has a redox sensor-like function triggering oligomerization and crosslinking upon oxidative stress related to cell death, Cell Death Dis. 2015 Jan 22;6(1):e1616].

We previously found that RITA induced the formation of a covalent link between two oxidized Trp114 residues of two subunits from two separate TrxRl dimers, as found both in cell extracts and in a crystal structure of tetrameric TrxR1. Formation of covalently linked TrxRl subunits was in direct correlation with triggering of a cell death that could be prevented by antioxidant treatment.

Using the same approach as in our publication [Xu J, Eriksson SE, Cebula M, Sandalova T, Hedström E, Pader I, Cheng Q, Myers CR, Antholine WE, Nagy P, Hellman U, Selivanova G, Lindqvist Y, Arner ES. The conserved Trp 1 14 residue of thioredoxin reductase 1 has a redox sensor-like function triggering oligomerization and crosslinking upon oxidative stress related to cell death, Cell Death Dis. 2015 Jan 22;6(1): e1616], we tested whether **1d*HCl** acts in a similar fashion. We tested whether **1d*HCl** can facilitate the formation of covalent dimers of TrxRl using Western blot. Thereby it was shown that 1d*HCl induces the formation of TrxR1 covalent dimers, as indicated by the appearance of a 130 kDa band detected by anti-TrxR1 antibody, thus corresponding to the covalent TrxR1 dimer, upon treatment of HCT116 and MCF7 cells with 5 µM of **1d*HCl**. The dimer formation was detected as soon as 2h after treatment, suggesting a fast kinetics.

### 1d*HCl does not induce DNA damage in cancer cells

The majority of chemotherapeutic drugs used currently in the clinic, are DNA damaging drugs. Induction of DNA damage is a very efficient way of elimination of cancer cells. Unfortunately, these drugs induce DNA damage also in normal cells, leading to severe side effects. We addressed the question whether 1**d*HCl** induces DNA damage. We tested this by detection of a hallmark of DNA damage, induction of phosphorylated histone H2AX, so called γH2AX. We used anti-γH2AX antibody to detect it in cells treated with **1d*HCl**. It was found that the induction of γH2AX by **1d*HCl** in two cancer cell lines, HCT116 and MCF7, was negligible. In addition, we did not detect a significant induction of p53, another sign of DNA damage. Thus, we conclude that **1d*HCl** does not induce DNA damage, which suggests that it will not induce in patients side effects associated with DNA damage.

### TrxR1 inhibition is a major mechanism of action of 1d*HCl

To find out whether the inhibition of TrxR1 is the major mechanism of action of **1d*HCl**, we addressed the question, whether stimulation of TrxRl activity will decrease the growth suppression activity of **1d*HCl**. Since TrxR is a selenium-containing enzyme and the concentration of selenium is rate-limiting for TrxRl, we added selenium to culture medium to test whether selenium will alleviate the growth suppression by **1d*HCl**. Thereby it was shown that the growth suppression by **1d*HCl** is less efficient in the presence of selenium in culture medium.

Further, we tested whether the inhibition of GR by BSO will have additive effect with **1d*HCl**. If **1d*HCl** mechanism of action includes inhibition of GR, BSO will not have an additive effect. We used a range of concentrations of **1d*HCl** and BSO in cell growth assays. It has been shown that already 5 µM of **1d*HCl** and the lowest concentration of BSO - 50 µM have an additive effect and completely eradicated cancer cells. This confirms our results that **1d*HCl** selectively inhibits TrxRl, but not GR.

Next, we tested whether the prevention of oxidative stress by NAC will affect the growth suppression by the combination of **1d*HCl** and BSO. In the presence of NAC we observed the additive effect of **1d*HCl** with 50 µM BSO only at twice higher dose of **1d*HCl** - 10 µM. These data are in line with our results shown above that NAC prevents the effects of **1d*HCl**.

Taken together, these results provide strong evidence that the inhibition of TrxRl by **1d*HCl** is the major mechanism of action of **1d*HCl**.

### 1d*HCl is more potent in killing the cells under hypoxia condition: Implications for treating hypoxic solid tumors

Hypoxic tumors are often the most aggressive and resistant to chemotherapy. Therefore, we tested how cells in hypoxia will respond to the treatment by **1d*HCl**. It has been shown that **1d*HCl** is even more efficient in hypoxia condition. IC₅₀ of **1d*HCl** in cancer cells grown in hypoxia is 2.3-fold lower than that in normoxia. Thus, the compounds of the present invention are of great interest for the treatment of hypoxic solid tumors.

### Anti-tumor activity of 1d*HCl in vivo

We tested the anti-tumor activity of **1d*HCl** using allogenic model of B16 mice. We inoculated mice with B 16 melanoma cells. When the tumors reached 100 mm³, we treated them with i.p. injection of **1d*HCl,** 10mg/kg every other day for 9 days. Mice that got i.p. injections of **1d*HCl** had lower volume of tumors on day 8 and on day 9 compared to vehicle-treated mice average tumor volume of 5.3 ± 2.7 mm³. The differences in tumor volume between untreated control mice and animals treated with **1d*HCl** are all statistically significant. Thus, **1d*HCl** has *in vivo* anti-tumor activity in this animal tumor model.

## Claims

1. A compound of formula (I): 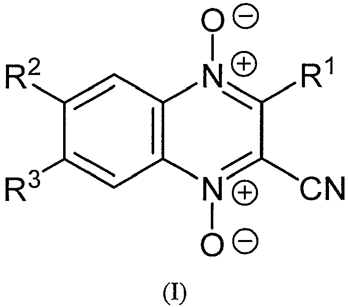 wherein
R¹ is a 2-pyridyl group, a 3-pyridyl group or a 4-pyridyl group, all of which groups may optionally be substituted by 1, 2 or 3 substituents that are independently selected from a C₁₋₆ alkyl group, a C₁₋₆ alkoxy group, a Ci-6 alkylthio group and a halogen atom;
R² is a group of formula -NR⁴R⁵, and R³ is a halogen atom; or
R² is a halogen atom, and R³ is a group of formula -NR⁴R⁵;
R⁴ is a C₁₋₆ alkyl group; and
R⁵ is a C₁₋₆ alkyl group; or
R⁴ and R⁵ together with the nitrogen atom to which they are bound form a heterocycloalkyl group comprising 5 or 6 ring atoms that are independently selected from C, N, S and O, which heterocycloalkyl group may optionally be substituted by 1, 2 or 3 substituents that are independently selected from a C₁₋₆ alkyl group, a C₁₋₆ alkoxy group, a hydroxy C₁₋₆ alkyl group and a hydroxy group;
or a salt, solvate or hydrate thereof;
wherein the expression "C₁₋₆ alkyl" refers to a saturated, straight-chain or branched or cyclic hydrocarbon group that contains from 1 to 6 carbon atoms.

2. A compound according to claim 1, wherein R² is a group of formula -NR⁴R⁵ and R³ is a fluorine atom.

3. A compound according to claim 1, wherein R² is a fluorine atom and R³ is a group of formula -NR⁴R⁵.

4. A compound according to any one of the preceding claims, wherein R¹ is an unsubstituted 2-pyridyl group, an unsubstituted 3-pyridyl group or an unsubstituted 4-pyridyl group.

5. A compound according to any one of the preceding claims, wherein the group of formula -NR⁴R⁵ is selected from the group consisting of morpholine, thiomorpholine, piperidine and pyrrolidine, all of which groups may optionally be substituted by 1, 2 or 3 substituents that are independently selected from a C₁₋₆ alkyl group, a C₁₋₆ alkoxy group, a hydroxy C₁₋₆ alkyl group and a hydroxy group.

6. A compound according to any one of the preceding claims 1 to 4, wherein R⁴ is a C₁₋₆ alkyl group and R⁵ is a C₁₋₆ alkyl group.

7. A compound according to claim 1 which is selected from the following compounds: 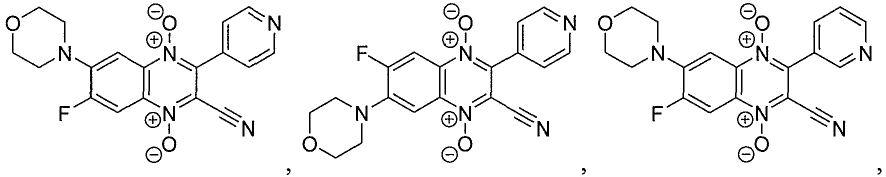 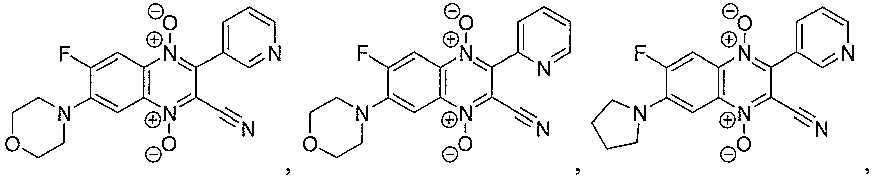 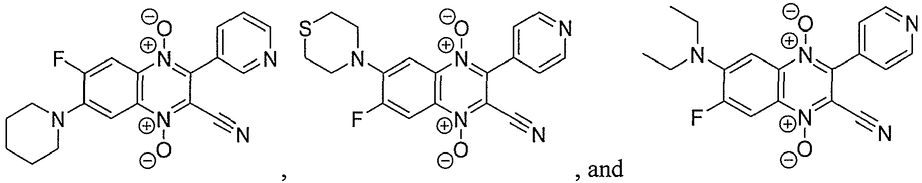 or a salt, solvate or hydrate thereof.

8. A pharmaceutical composition comprising a compound according to anyone of the preceding claims and optionally one or more carrier substances and/or one or more adjuvants.

9. A compound according to any one of claims 1 to 7 or a pharmaceutical composition according to claim 8 for use in the treatment of cancer.

10. A compound according to any one of claims 1 to 7 or a pharmaceutical composition according to claim 8 for use in the treatment of carcinomas of breast, lung, colon, liver, ovary and cervix, sarcoma, melanoma, leukemia, lymphoma, and neuroblastoma.

## Patentansprüche

1. Verbindung der Formel (I): 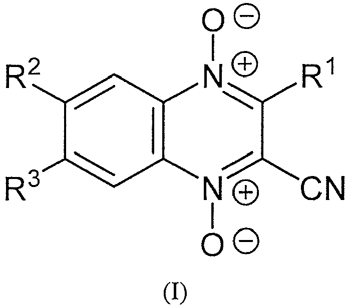 wobei
R¹ eine 2-Pyridylgruppe, eine 3-Pyridylgruppe oder eine 4-Pyridylgruppe ist, wobei alle diese Gruppen optional durch 1, 2 oder 3 Substituenten substituiert sein können, die unabhängig voneinander aus einer C₁₋₆ Alkylgruppe, einer C₁₋₆ Alkoxygruppe, einer C₁₋₆ Alkylthiogruppe und einem Halogenatom ausgewählt sind;
R² eine Gruppe der Formel -NR⁴R⁵, und R³ ein Halogenatom ist; oder
R² ein Halogenatom, und R³ eine Gruppe der Formel -NR⁴R⁵ist;
R⁴ eine C₁₋₆ Alkylgruppe ist; und
R⁵ eine C₁₋₆ Alkylgruppe ist; oder
R⁴ und R⁵ zusammen mit dem Stickstoffatom, an das sie gebunden sind, eine Heterocycloalkylgruppe bilden, die 5 oder 6 Ringatome umfasst, die unabhängig voneinander aus C, N, S und O ausgewählt sind, wobei die Heterocycloalkylgruppe optional durch 1, 2 oder 3 Substituenten substituiert sein kann, die unabhängig voneinander aus einer C₁₋₆ Alkylgruppe, einer C₁₋₆ Alkoxygruppe, einer Hydroxy-C₁₋₆ Alkylgruppe und einer Hydroxygruppe ausgewählt sind;
oder ein Salz, Solvat oder Hydrat davon
wobei sich der Ausdruck "C₁₋₆ Alkyl" auf eine gesättigte, geradkettige oder verzweigte oder cyclische Kohlenwasserstoffgruppe bezieht, die 1 bis 6 Kohlenstoffatome enthält.

2. Verbindung nach Anspruch 1, wobei R² eine Gruppe der Formel -NR⁴R⁵ist und R³ ein Fluoratom ist.

3. Verbindung nach Anspruch 1, wobei R² ein Fluoratom ist und R³ eine Gruppe der Formel -NR⁴R⁵ist.

4. Verbindung nach einem der vorhergehenden Ansprüche, wobei R¹ eine unsubstituierte 2-Pyridylgruppe, eine unsubstituierte 3-Pyridylgruppe oder eine unsubstituierte 4-Pyridylgruppe ist.

5. Verbindung nach einem der vorhergehenden Ansprüche, wobei die Gruppe der Formel -NR⁴R⁵ausgewählt ist aus der Gruppe, bestehend aus Morpholin, Thiomorpholin, Piperidin und Pyrrolidin, wobei alle diese Gruppen optional durch 1, 2 oder 3 Substituenten substituiert sein können, die unabhängig voneinander aus einer C₁₋₆ Alkylgruppe, einer C₁₋₆ Alkoxygruppe, einer Hydroxy-Ci-6 Alkylgruppe und einer Hydroxygruppe ausgewählt sind.

6. Verbindung nach einem der vorhergehenden Ansprüche 1 bis 4, wobei R⁴ eine C₁₋₆-Alkylgruppe und R⁵ eine C₁₋₆-Alkylgruppe ist.

7. Verbindung nach Anspruch 1, die aus den folgenden Verbindungen ausgewählt ist: 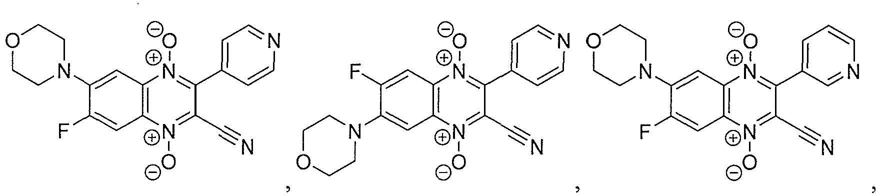 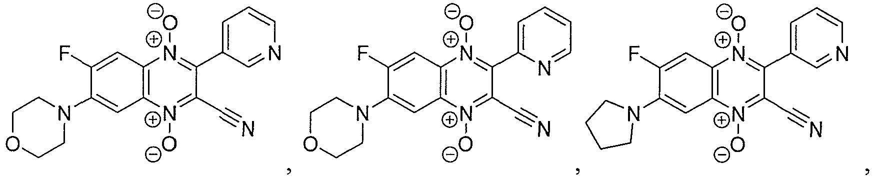 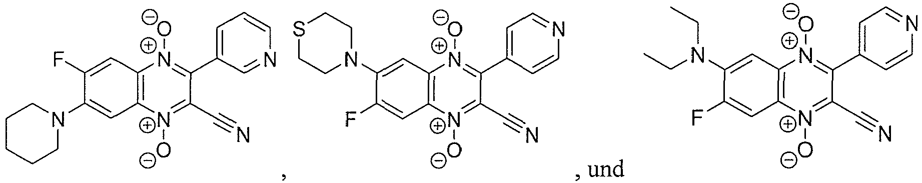 oder ein Salz, Solvat oder Hydrat davon.

8. Pharmazeutische Zusammensetzung, die eine Verbindung nach einem der vorhergehenden Ansprüche und optional eine oder mehrere Trägersubstanzen und/oder ein oder mehrere Adjuvantien enthält.

9. Verbindung nach einem der Ansprüche 1 bis 7 oder eine pharmazeutische Zusammensetzung nach Anspruch 8 zur Verwendung bei der Behandlung von Krebs.

10. Verbindung nach einem der Ansprüche 1 bis 7 oder eine pharmazeutische Zusammensetzung nach Anspruch 8 zur Verwendung bei der Behandlung von Karzinomen der Brust, der Lunge, des Dickdarms, der Leber, des Eierstocks und des Gebärmutterhalses, Sarkomen, Melanomen, Leukämie, Lymphomen und Neuroblastomen.

## Revendications

1. Composé de formule (I): 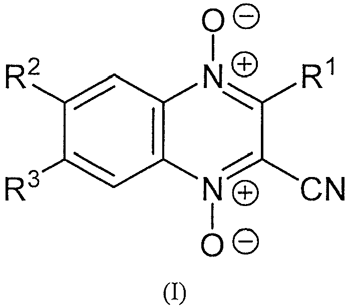 dans lequel
R¹ est un groupe 2-pyridyle, un groupe 3-pyridyle ou un groupe 4-pyridyle, tous ces groupes pouvant être éventuellement substitués par 1, 2 ou 3 substituants choisis indépendamment parmi un groupe alkyle en C₁₋₆, un groupe alcoxy en C₁₋₆, un groupe alkylthio en C₁₋₆ et un atome d'halogène;
R² est un groupe de formule -NR⁴R⁵, et R³ est un atome d'halogène; ou
R² est un atome d'halogène, et R³ est un groupe de formule -NR⁴R⁵;
R⁴ est un groupe alkyle en C₁₋₆ ; et
R⁵ est un groupe alkyle en C₁₋₆; ou
R⁴ et R⁵ forment, avec l'atome d'azote auquel ils sont liés, un groupe hétérocycloalkyle comprenant 5 ou 6 atomes d'anneau choisis indépendamment parmi C, N, S et O, ce groupe hétérocycloalkyle pouvant éventuellement être substitué par 1, 2 ou 3 substituants choisis indépendamment parmi un groupe alkyle en C₁₋₆, un groupe alcoxy en C₁₋₆ , un groupe alkyle en C₁₋₆ hydroxy et un groupe hydroxy;
ou un sel, un solvant ou un hydrate de celui-ci,
où l'expression "alkyl en C₁₋₆" désigne un groupe d'hydrocarbures saturé, linéaire ou ramifié ou cyclique, contenant de 1 à 6 atomes de carbone.

2. Composé selon la revendication 1, dans lequel R² est un groupe de formule -NR⁴R⁵ et R³ est un atome de fluor.

3. Composé selon la revendication 1, dans lequel R² est un atome de fluor et R³ est un groupe de formule -NR⁴R⁵.

4. Composé selon l'une des revendications précédentes, dans lequel R¹ est un groupe 2-pyridyle non substitué, un groupe 3-pyridyle non substitué ou un groupe 4-pyridyle non substitué.

5. Composé selon l'une des revendications précédentes, dans lequel le groupe de formule -NR⁴R⁵ est choisi parmi le groupe constitué par la morpholine, la thiomorpholine, la pipéridine et la pyrrolidine, tous ces groupes pouvant éventuellement être substitués par 1, 2 ou 3 substituants choisis indépendamment parmi un groupe alkyle en C₁₋₆ , un groupe alcoxy en C₁₋₆ , un groupe alkyle en C₁₋₆ hydroxy et un groupe hydroxy.

6. Composé selon l'une des revendications précédentes 1 à 4, dans lequel R⁴ est un groupe alkyle en C₁₋₆ et R⁵ est un groupe alkyle en C₁₋₆

7. Composé selon la revendication 1 qui est choisi parmi les composés suivants : 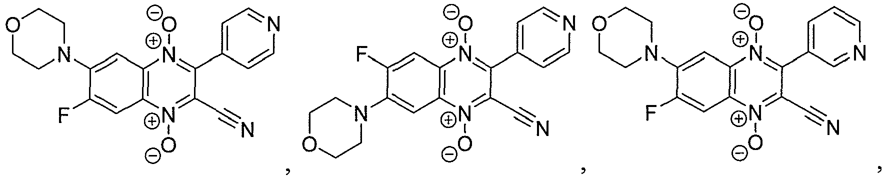 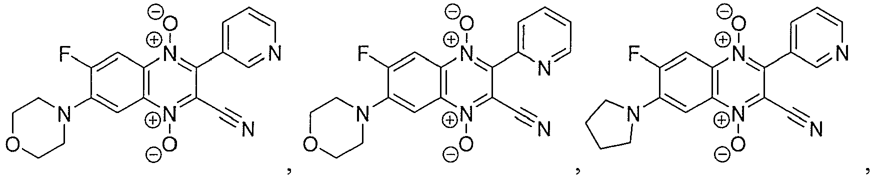 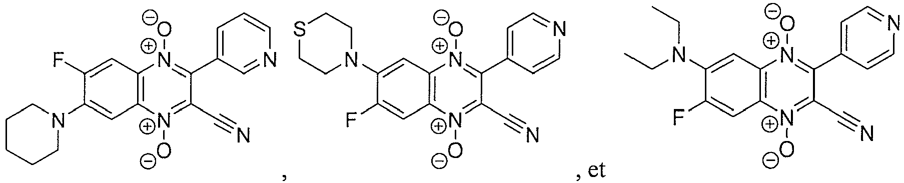 ou un sel, un solvant ou un hydrate de celui-ci.

8. Composition pharmaceutique comprenant un composé selon l'une des revendications précédentes et éventuellement une ou plusieurs substances porteuses et/ou un ou plusieurs adjuvants.

9. Composé selon l'une des revendications 1 à 7 ou composition pharmaceutique selon la revendication 8 pour utilisation dans le traitement du cancer.

10. Composé selon l'une des revendications 1 à 7 ou composition pharmaceutique selon la revendication 8 pour utilisation dans le traitement des carcinomes du sein, du poumon, du colon, du foie, de l'ovaire et du col de l'utérus, des sarcomes, des mélanomes, des leucémies, des lymphomes et des neuroblastomes.
